# EUROPÄISCHE PATENTSCHRIFT

(11) **EP 1 985 258 B1**
(45) Veröffentlichungstag und Bekanntmachung des Hinweises auf die Patenterteilung: **27.11.2013**
(21) Anmeldenummer: 08007336.4
(22) Anmeldetag: 14.04.2008
(51) Int. Cl.: A61F 2/07

(54) **Gefäßimplantat zur Behandlung eines Aneurysmas**
Vascular implant for treating an aneurysm
Implant vasculaire destiné au traitement d'un anévrisme

(30) Priorität: 23.04.2007 DE 102007019058
(43) Veröffentlichungstag der Anmeldung: 29.10.2008
(73) Patentinhaber: Stengel, Max, 76646 Bruchsal (DE)
(72) Erfinder: Stengel, Max, 76646 Bruchsal (DE)
(74) Vertreter: Manitz, Finsterwald & Partner GbR

(56) Entgegenhaltungen:
- EP-A2- 1 698 302
- US-A- 5 609 627
- US-A1- 2003 149 472
- US-A1- 2003 236 567
- US-A1- 2004 049 258
- US-A1- 2006 195 172
- US-A1- 2006 287 704

## Beschreibung

Die vorliegende Erfindung betrifft ein Gefäßimplantat zur Behandlung eines Aneurysmas gemäß dem Oberbegriff des Anspruchs 1. Ein derartiges Gefäßimplantat ist aus der US 2006/0287704 A1 bekannt.

Bei einem Aneurysma handelt es sich um eine krankhafte, örtlich begrenzte Erweiterung, Ausbuchtung bzw. Aussackung eines Blutgefäßes, insbesondere einer Schlagader. Aneurysmata können an sämtlichen Abschnitten einer Schlagader auftreten. Besonders häufig sind Bauchaortenaneurysmata und Aneurysmata an Kniekehlenschlagadern vorzufinden. Aufgeweitete Blutgefäße unterliegen einer hohen Rupturgefahr, so dass das Risiko einer tödlichen Blutung besteht. Darüber hinaus neigen aufgeweitete Blutgefäße dazu, an Größe zuzunehmen. Die Rupturgefahr steigt mit zunehmendem Durchmesser des Aneurysmas, da der durch das durch das Blutgefäß strömende Blut auf die Gefäßwand des Blutgefäßes ausgeübte Druck mit zunehmender Gefäßweite zunimmt (Gesetz von Bernoulli).

Es ist bekannt, derartig aufgeweitete Blutgefäße mit röhrenförmigen bzw. schlauchförmigen Implantaten, welche auch als Stents bezeichnet werden, zu versorgen. Hierbei wird ein Stent mit einer blutundurchlässigen Wand in das betroffene Blutgefäß derart eingesetzt, dass mittels des Stents das Aneurysma überbrückt werden kann. Dabei wird der Stent mit seinen beiden Enden an den an den aufgeweiteten Bereich beidseitig anschließenden, nicht aufgeweiteten Bereichen des Blutgefäßes möglichst formschlüssig fixiert, wodurch eine Abdichtung der Aufweitung erreicht werden soll, um zu verhindern, dass weiterhin Blut in die Aufweitung fließt. Der Durchmesser eines derartigen Stents entspricht in etwa dem normalen Durchmesser eines Gefäßes.

Bei diesen Lösungen besteht jedoch das Problem, dass durch ungenügende Adaption an das natürliche Gefäß häufig kleine Undichtigkeiten zwischen dem Stent und demjenigen Bereich der Gefäßwand des Blutgefäßes, an welchem der Stent fixiert ist, entstehen können. Insbesondere durch eintrittsöffnungsseitig vorhandene Undichtigkeiten oder durch Kollateralgefäße kann weiterhin Blut in das Aneurysma strömen, so dass die Rupturgefahr der Gefäßwand im Bereich des Aneurysmas nicht beseitigt wird.

Der Erfindung liegt daher die Aufgabe zugrunde, ein Gefäßimplantat der eingangs genannten Art anzugeben, welches die Gefahr einer Ruptur der Gefäßwand im Bereich eines Aneurysmas verringert.

Diese Aufgabe wird durch ein Gefäßimplantat mit den Merkmalen des Anspruchs 1 gelöst und insbesondere dadurch, dass die Eintrittsöffnung eine größere Querschnittsfläche besitzt als die Austrittsöffnung.

Bevorzugt wird das Gefäßimplantat mit dem die Eintrittsöffnung aufweisenden Ende und/oder einem an die Eintrittsöffnung unmittelbar anschließenden Abschnitt an dem stromaufwärts des Aneurysmas gelegenen, nicht aufgeweiteten Bereich des Blutgefäßes fixiert. Bevorzugt besitzt das Gefäßimplantat eine Länge, welche sich über die gesamte Länge des Aneurysmas hinweg erstrecken kann.

Nach dem Kontinuitätsgesetz für inkompressible Fluide tritt aus der Austrittsöffnung des Körpers dasselbe Blutvolumen aus, das an der Eintrittsöffnung des Körpers in diesen eingeströmt ist, d.h. der Volumenstrom bzw. das Volumen, das sich innerhalb einer Zeiteinheit durch einen Querschnitt des Körpers bewegt, ist an der Eintrittsöffnung und an der Austrittsöffnung der- bzw. dasselbe. Hieraus ergibt sich, dass das durch das Blutgefäß und damit den Körper strömende Blut die Austrittsöffnung mit einer höheren Geschwindigkeit passiert als die Eintrittsöffnung. Durch die höhere Geschwindigkeit entsteht an dem die Austrittsöffnung aufweisenden Ende des Körpers - wie bei einer Wasserstrahlpumpe - ein Unterdruck, durch den das in einem Aneurysma befindliche Blut abgesaugt wird. Auf diese Weise entsteht auch innerhalb des Aneurysmas ein entsprechender Unterdruck, so dass die aufgeweitete Gefäßwand entlastet wird.

Dieser Effekt tritt selbst dann auf, wenn das Gefäßimplantat eintrittsöff nungsseitig nicht vollständig abgedichtet ist. Somit kann mit dem erfindungsgemäß ausgebildeten Gefäßimplantats die Gefahr einer Ruptur der Gefäßwand des Aneurysmas wirksam verringert werden.

Da eine stromabwärtsseitige Abdichtung des Aneurysmas durch das Gefäßimplantat nicht erforderlich ist und selbst stromaufwärts die Abdichtung nicht 100%ig sein muss, kann das Gefäßimplantat auch in solchen Fällen verwendet werden, bei denen eine abdichtende Fixierung an dem Blutgefäß stromaufwärts und/oder stromabwärts des Aneurysmas nicht möglich ist. Da stromabwärts des Aneurysmas eine Abdichtung nicht erforderlich ist, kann das erfindungsgemäße Gefäßimplantat auch bei einem Bauchaortenaneurysma eingesetzt werden, welches unmittelbar an der Abzweigung zu den beiden Beinschlagadern beginnt. Auf eine Fixierung stromabwärts des Aneurysmas kann entweder verzichtet werden oder es kann eine blutdurchlässige Fixierung verwendet werden. Für ein Bauchaortenaneurysma, welches unmittelbar an den Abzweigungen zu den beiden Nierenschlagadern beginnt, kann das Gefäßimplantat mit einem an die Eintrittsöffnung des Körpers insbesondere unmittelbar anschließenden Fixierabschnitt versehen sein, welcher blutdurchlässig ausgebildet ist, um die Abzweigungen zu den Nierenschlagadern nicht zu verschließen.

Nach einer Ausbildung der Erfindung verjüngt sich der Körper, ein zwischen der Eintrittsöffnung und der Austrittsöffnung befindlicher Abschnitt des Körpers und/oder der Durchgangskanal von der Eintrittsöffnung zur Austrittsöffnung hin. Hierdurch kann über die gesamte Länge des Körpers eine allmähliche Querschnittsflächenreduzierung von der Eintrittsöffnung zur Austrittsöffnung erreicht werden, um beispielsweise Verwirbelungen im Blutstrom zu vermeiden. Die Verjüngung ist bevorzugt gleichmäßig, kontinuierlich und/oder konisch ausgebildet.

Bevorzugt ist der Körper im Bereich der Eintrittsöffnung und/oder an einem an die Eintrittsöffnung anschließenden Abschnitt des Körpers zur Anlage an der Gefäßwand des Blutgefäßes ausgebildet. Die Außenquerschnittsfläche des Körpers im Bereich der Eintrittsöffnung und/ oder des an die Eintrittsöffnung anschließenden Abschnitts ist insbesondere derart dimensioniert, dass im implantierten Zustand der Körper im Bereich der Eintrittsöffnung und/oder mit dem an die Eintrittsöffnung anschließenden Abschnitt an der Gefäßwand des Blutgefäßes anliegt. Hierdurch kann eine Fixierung des Körpers im Blutgefäß erreicht werden.

Um eine besonders gute Fixierung des Gefäßimplantats im Blutgefäß zu erreichen, kann es vorteilhaft sein, wenn der an die Eintrittsöffnung anschließende Abschnitt des Körpers zylinderförmig ausgebildet ist. Der Körper kann somit über einen größeren Bereich hinweg an den Innendurchmesser des Blutgefäßes adaptiert werden.

Besonders bevorzugt ist es, wenn der an die Eintrittsöffnung anschließende Abschnitt des Körpers eine blutdurchlässig ausgebildete Wand besitzt. Der an die Eintrittsöffnung anschließende Abschnitt kann beispielsweise als Gittergeflecht ausgebildet sein. Hierdurch wird ein Einwachsen der Zellen der Gefäßwand des Blutgefäßes in den Körper des Implantats gefördert und damit eine besonders feste Fixierung geschaffen.

Nach einer weiteren Ausbildung der Erfindung ist die äußere Abmessung des Körpers im Bereich der Austrittsöffnung derart dimensioniert, dass der Körper im Bereich der Austrittsöffnung von der Gefäßwand des Blutgefäßes beabstandet ist. Dies kann insbesondere dann der Fall sein, wenn sich neben der Querschnittsfläche des Durchgangskanals auch die äußere Abmessung des Körpers zu der die Austrittsöffnung aufweisenden Seite hin verjüngt.

Der Körper ist mit blutdurchlässigen Fixiermitteln für das austrittsöffnungsseitige Ende des Körpers versehen welche zur Anlage an der Gefäßwand des Blutgefäßes ausgebildet sind. Hierdurch kann ein seitliches Hin- und Herwandern des die Austrittsöffnung aufweisenden Endes des Körpers in der Blutströmung verhindert werden, so dass eine radiale Fixierung innerhalb des Blutgefäßes erreicht wird. Die Fixiermittel sind blutdurchlässig ausgebildet, um ein durch den erzeugten Unterdruck bewirktes Absaugen des Bluts aus dem Aneurysma möglichst wenig zu behindern.

Die Fixiermittel sind dazu ausgebildet, den Körper im Bereich der Austrittsöffnung von der Gefäßwand des Blutgefäßes beabstandet zu halten Hierdurch wird gewährleistet, dass das die Austrittsöffnung aufweisende Ende des Körpers nicht an der Gefäßwand des Blutgefäßes anliegt, so dass der vorstehend erläuterte Wasserstrahlpumpeneffekt vollumfänglich um die Austrittsöffnung herum auftreten kann. Insbesondere können die Fixiermittel derart ausgebildet sein, dass die Austrittsöffnung oder das die Austrittsöffnung aufweisende Ende des Körpers zentriert innerhalb des Blutgefäßes gehalten wird.

Nach einer weiteren Ausbildung der Erfindung sind die Fixiermittel im Bereich der Austrittsöffnung an dem Körper vorgesehen.

Die Fixiermittel können als ein Gittergeflecht, mehrere jeweils mit einer radialen Komponente von dem Körper abstehende Fixierelemente und/oder ein konischer Stent ausgebildet sein.

Der Körper oder ein Teil hiervon kann mit den Fixiermitteln einteilig ausgebildet sein. Die Fixiermittel können jedoch auch als separates Teil oder als separate Teile ausgebildet sein, das/die mit dem Körper oder einem Teil hiervon verbunden ist/ sind.

Die Wand des Körpers kann starr, flexibel oder teils starr und flexibel ausgebildet sein. Bei der Wand kann es sich beispielsweise um ein Gittergeflecht in Form eines Stents handeln, welches mit einer Beschichtung versehen ist. Die Beschichtung kann dabei an der Außenseite, an der Innenseite oder sowohl an der Außenseite wie auch an der Innenseite des Gittergeflechts vorgesehen sein. Bevorzugt ist die Wand aus biokompatiblem Material hergestellt. Beispielsweise kann das Gittergeflecht als selbstexpandierender oder als ballonexpandierender Stent ausgebildet sein. Während bei einer starren Ausbildung das Gefäßimplantat beispielsweise operativ in das Blutgefäß eingesetzt wird, ist bei einer zumindest teilflexiblen Ausbildung eine transluminale Implantation möglich.

Das Gefäßimplantat, insbesondere der Körper oder die Wand des Körpers, kann mehrteilig aufgebaut sein. In diesem Fall können die einzelnen Teile getrennt voneinander in das Blutgefäß eingebracht und/oder innerhalb des Blutgefäßes abgesetzt werden. Beispielsweise kann bei einer mehrteilig ausgebildeten Wand, welche eine blutundurchlässige Hülle und eine Stützstruktur für die Hülle, beispielsweise ein Gittergeflecht, umfasst, die Hülle getrennt von der Stützstruktur und/oder getrennt von Fixiermitteln für den Körper in das Blutgefäß eingebracht und/oder innerhalb des Blutgefäßes abgesetzt werden. Bei einer getrennten Einbringung, beispielsweise hintereinander liegend auf einem Katheter oder aufeinanderfolgend jeweils mit unterschiedlichen Kathetern, kann der Zugang für den Katheter kleiner gewählt werden, so dass die Wundheilung verbessert wird.

Es ist beispielsweise möglich, dass zunächst lediglich die Hülle eingebracht und abgesetzt wird, wobei diese bevorzugt nach dem Absetzen an einem Ende durch das Einführinstrument gehalten wird. Anschließend kann die Stützstruktur in die Hülle eingesetzt werden, so dass beim Aufweiten der Stützstruktur gleichzeitig die Hülle mit aufgeweitet wird und diese durch die Stützstruktur gegen die Gefäßwand gedrückt und durch gehalten wird. Falls gewünscht, können in einem nächsten Schritt die Fixiermittel eingebracht und mit dem aus Stützstruktur und Hülle gebildeten Körper verbunden werden.

Die Wand kann auch lediglich eine blutundurchlässige Hülle, insbesondere ohne eine Stützstruktur, umfassen. Die Hülle kann dann beispielsweise an einem an die Eintrittsöffnung stromaufwärts anschließenden, insbesondere sondere von der Hülle getrennt ausgebildeten Abschnitt des Körpers befestigt sein oder von diesem zur Befestigung gegen die Gefäßwand gedrückt werden. In diesem Fall besitzt die Stützstruktur bevorzugt eine kurze Länge, so dass sie nur stromaufwärts des Aneurysmas zu liegen kommt oder nur über einen insbesondere relative kurzen Teilbereich in das Aneurysma hineinragt.

Die vorliegende Erfindung betrifft weiterhin ein Gefäßimplantat, mit zwei Körpern, welche jeweils wie vorstehend erläutert ausgebildet sind, wobei die Körper an einer Abzweigung des Gefäßimplantats im Bereich ihrer Eingangsöffnungen miteinander verbunden sind. Ein derartiges Gefäßimplantat kann beispielsweise bei einem Bauchaortenaneurysma im Bereich der Abzweigung zu den beiden Beinschlagadern verwendet werden.

Insbesondere können die beiden Körper einen an die Abzweigung anschließenden gemeinsamen Abschnitt zur Anlage an der Gefäßwand des Blutgefäßes aufweisen.

Nicht beschränkende Ausführungsbeispiele der Erfindung sind in der Zeichnung dargestellt und werden nachfolgend beschrieben.

Es zeigen, jeweils in schematischer Darstellung,
- Fig.1: ein erste Ausführungsform eines Gefäßimplantats,
- Fig. 2: das Gefäßimptantat aus Fig. 1 mit erfindungsgemäßen Fixierelementen,
- Fig. 3: das Gefäßimplantat aus Fig. 1 mit erfindungsgemäßen sternförmigen Fixiermitteln,
- Fig. 4: das Gefäßimplantat aus Fig. 1 mit erfindungsgemäßen als konischen Stent ausgebildeten Fixiermitteln, und
- Fig. 5: ein weitere Ausführungsform eines Gefäßimplantats.

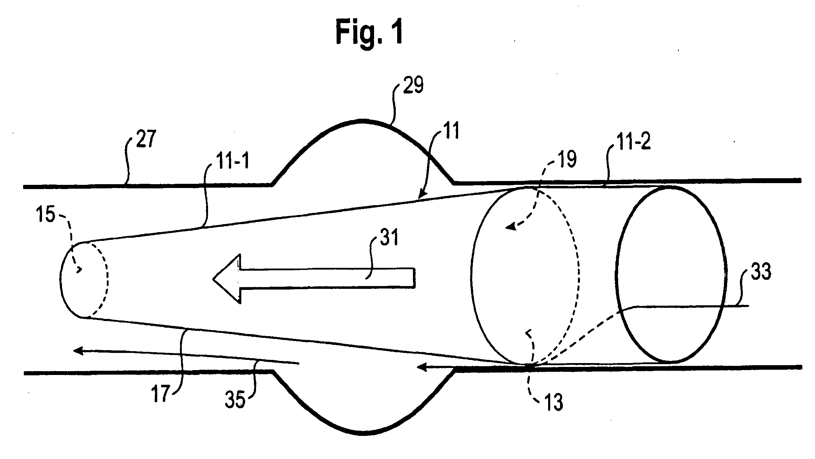

Das in Fig. 1 gezeigte Gefäßimplantat umfasst einen länglichen, röhrenförmigen Körper 11, welcher in einen Abschnitt 11-1 und einen Abschnitt 11-2 aufgeteilt ist. Der Abschnitt 11-1 des Körpers 11 ist konisch ausgebildet und weist eine blutundurchlässige Wand 17 auf. In Längsrichtung ist der Abschnitt 11-1 durch eine Eintrittsöffnung 13 und eine Austrittsöffnung 15 begrenzt, welche durch einen konischen Durchgangskanal 19 miteinander verbunden sind, der in radialer Richtung durch die Wand 17 begrenzt ist. Der zweite Abschnitt 11-2 ist zylinderförmig ausgebildet und schließt eintrittsöffnungsseitig unmittelbar an den ersten Abschnitt 11-1 des Körpers 11 an. Der zweite Abschnitt 11-2 ist jedoch optional und kann auch weggelassen werden.

Die Eintrittsöffnung 13 besitzt eine größere Querschnittsfläche als die Austrittsöffnung 15, d.h. der innere Durchmesser bzw. das Lumen der Eintrittsöffnung 13 ist größer als der innere Durchmesser bzw. das Lumen der Austrittsöffnung 15.

Das Gefäßimplantat mit dem Körper 11 ist in ein Blutgefäß 27 eingesetzt, welches lokal eine Querschnittsflächenaufweitung 29, d.h. ein Aneurysma, aufweist. Der Körper 11 liegt mit seinem zylinderförmigen Abschnitt 11-2 und im Bereich der Eintrittsöffnung 13 in einem nicht aufgeweiteten Bereich des Blutgefäßes 27 an dessen Gefäßwand an, wobei der Außendurchmesser des zylinderförmigen Abschnitts 11-2 und der Außendurch-messer im Bereich der Eintrittsöffnung 13 geringfügig größer gewählt sind als der Innendurchmesser des Blutgefäßes 27, so dass der zylinderförmige Abschnitt 11-2 des Körpers 11 unter Spannung an der Gefäßwand anliegt, wodurch eine feste Halterung des Gefäßimplantats erreicht wird. Der Abschnitt 11-2 ist als Gittergeflecht ausgebildet sein, um ein Einwachsen der Zellen der Gefäßwand des Blutgefäßes 27 zu fördern.

Die Festlegung des zylinderförmigen Abschnitts 11-2 kann in an sich bekannter Weise erfolgen. Beispielsweise kann der Körper 11 des Gefäßimplantats als insbesondere aus Nitinol bestehender, selbstexpandierender Stent ausgebildet sein, der in einem komprimierten Zustand mittels eines Katheters in das Blutgefäß 27 eingebracht wird und nach Absetzen aufgrund der Bluttemperatur automatisch in einen Zustand mit größerem Radius expandiert, so dass sich der zylinderförmige Abschnitt 11-2 an die Gefäßwand anlegt. Es ist auch möglich, dass der Körper 11 des Gefäßimplantats beispielsweise aus Edelstahl oder aus Federstahl besteht und insbesondere über einen Ballon aufgeweitet wird, bis der zylindrische Abschnitt 11-2 an der Gefäßwand zur Anlage kommt.

Austrittsöffnungsseitig mündet der Körper 11 frei im Blutgefäß 27. Die Länge des Körpers 11 reicht aus, um das Aneurysma 29 vollständig zu überspannen. Es ist grundsätzlich jedoch auch denkbar, dass sich die Austrittsöffnung 15 des Körpers 11 im Bereich des Aneurysmas 29 befindet, solange gewährleistet ist, dass aufgrund des beschriebenen Wasserstrahlpumpeneffekts innerhalb des Aneurysmas 29 ein Unterdruck erzeugt wird.

Durch das Blutgefäß 27 in Flussrichtung 31 strömendes Blut tritt über den zylindrischen Abschnitt 11-2 und durch die Eintrittsöffnung 13 in den konischen Abschnitt 11-1 des Körper 11 ein und verlässt diesen durch die Austrittsöffnung 15, wobei innerhalb des konischen Abschnitts 11-2 die Fließgeschwindigkeit aufgrund des sich kontinuierlich verengenden Durchgangskanals 19 stetig zunimmt.

Zwischen der Gefäßwand des Blutgefäßes 27 und dem die blutundurchlässige Wand 17 aufweisenden konischen Abschnitt 11-1 des Körpers 11 im Bereich der Eintrittsöffnung 13 durch eine Undichtigkeit gemäß einem Pfeil 33 hindurch tretendes Blut, welches sich in dem Aneurysma 29 sammelt, wird durch das mit erhöhter Geschwindigkeit aus der Austrittsöffnung 15 strömende Blut aufgrund des vorstehend erläuterten Wasserstrahlpumpeneffekts in Flussrichtung 31 gefördert, so dass innerhalb des Aneurysmas 29 ein Unterdruck entsteht und der Aufbau eines zu hohen Drucks auf die Gefäßwand des Blutgefäßes 27 im Bereich des Aneurysmas 29 verhindert werden kann. Die durch den Unterdruck zwischen dem Aneurysma 29 und dem die Austrittsöffnung 15 des Körpers 11 umgebenden Bereich des Blutgefäßes 27 auftretende Druckdifferenz ist durch einen Pfeil 35 veranschaulicht.

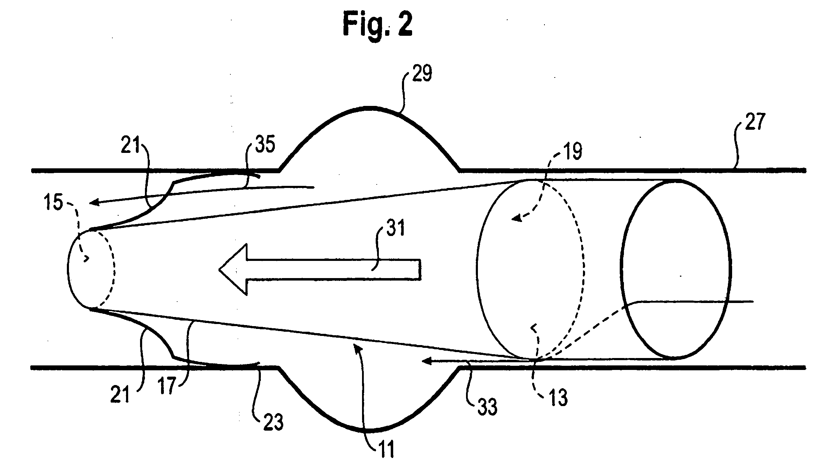

Das in Fig. 2 gezeigte Gefäßimplantat entspricht im Wesentlichen dem in Fig. 1 gezeigten Gefäßimplantat. Im Unterschied zu Fig. 1 weist das Gefäßimplantat gemäß Fig. 2 jedoch einen konischen Stent 21 mit blutdurchlässiger Wand auf, welcher sich in dieselbe Richtung wie der konische Abschnitt 11-1 des Körpers 11 verjüngt. Der einen größeren Öffnungswinkel als der konische Körperabschnitt 11-1 aufweisende konische Stent 21 ist mit seinem verjüngten Ende im Bereich der Austrittsöffnung 15 an dem Körper 11 angebracht. Der konische Stent 21 ist als Fixiermittel ausgebildet und stützt sich mit seinen dem Körper 11 abgewandten Endpunkten 23 an der Gefäßwand des Blutgefäßes 27 ab. Hierdurch wird der Körper 11 im Bereich der Austrittsöffnung 15 von der Gefäßwand des Blutgefäßes 27 beabstandet und zentriert innerhalb des Blutgefäßes 27 gehalten.

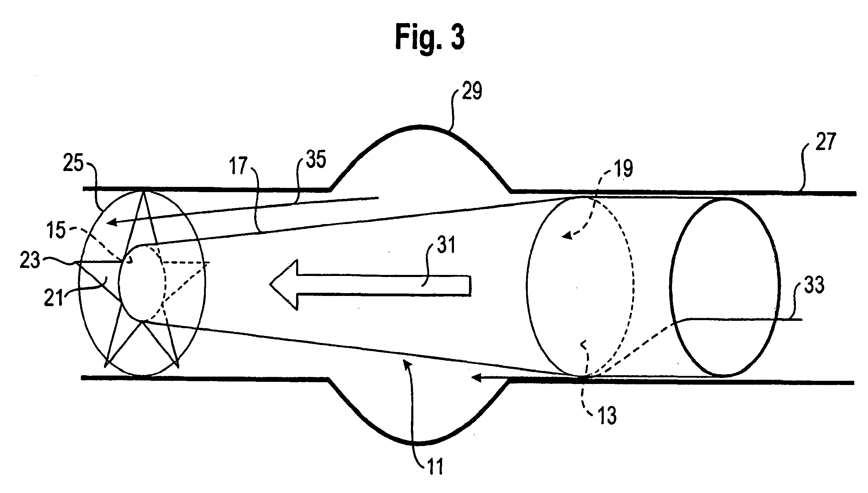

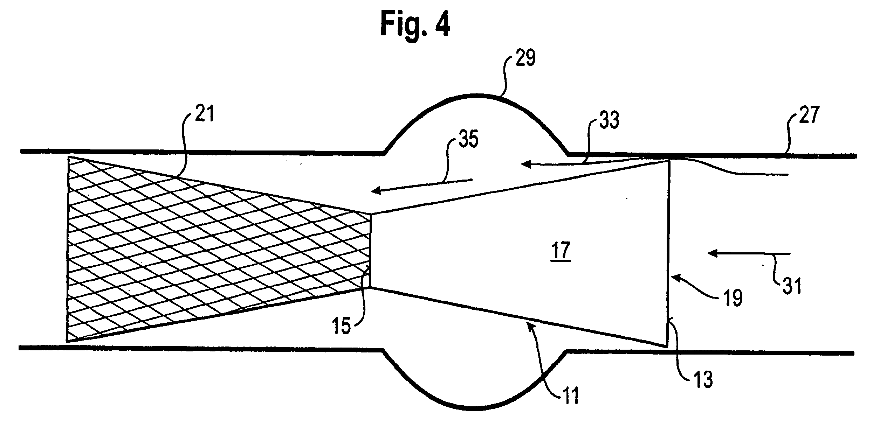

Die in Fig. 2 gezeigten Fixiermittel 21 sind jedoch lediglich beispielhafter Natur. Grundsätzlich können beliebig anders geformte blutdurchlässige Fixiermittel vorgesehen sein, welche das die Austrittsöffnung 15 aufweisende Ende des Körpers 11 von der Gefäßwand des Blutgefäßes 27 beabstandet und zentriert innerhalb des Blutgefäßes 27 halten können. Insbesondere können mehrere, radial nach außen abstehende Fixierelemente 21 vorgesehen sein. In Fig. 3 sind die Fixierelemente 21 beispielsweise in Form eines Sternes ausgebildet, dessen von dem Körper 11 abgewandte Endpunkte 23 auf einer gedachten Kreislinie 25 angeordnet sind, welche derart dimensioniert ist, dass die Endpunkte 23 an der Gefäßwand des Blutgefäßes 27 anliegen. Bei dem in Fig. 4 gezeigten Gefäßimplantat sind die Fixiermittel als blutdurchlässiger konischer Stent 21 ausgebildet, welcher aus einem Gittergeflecht besteht, so dass insgesamt ein doppelkegelstumpfförmiges Gefäßimplantat gebildet ist, welches mittig eine Einschnürung aufweist. Die in den Figuren 2 bis 4 gezeigten Fixiermittel 21 können mit dem Körper 11 jeweils einteilig ausgebildet oder im Bereich der Austrittsöffnung 15 mit dem Körper 11 verbunden sein.

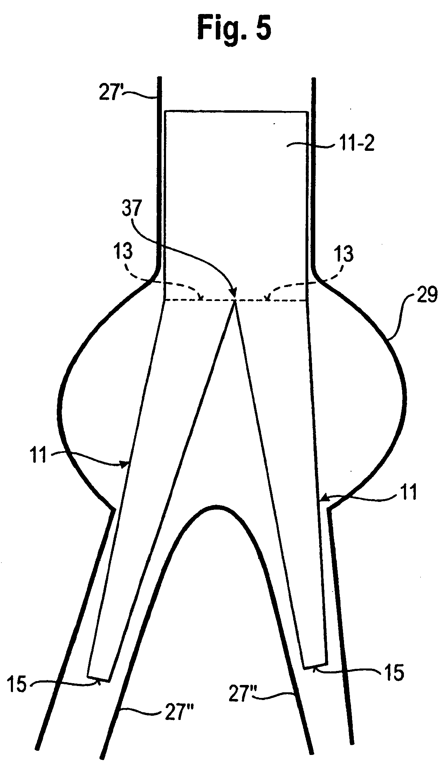

Das in Fig. 5 dargestellte Gefäßimplantat ist zumindest im Wesentlichen Y-förmig ausgebildet und umfasst zwei im Wesentlichen wie vorstehend beschriebene Körper 11, welche jeweils konisch ausgebildet sind. Die beiden Körper 11 sind im Bereich ihrer Eingangsöffnungen 13 an einer Abzweigung 37 des Gefäßimplantats miteinander verbunden, wobei für die beiden Körper 11 ein gemeinsamer zylindrischer Abschnitt 11-2 vorgesehen ist. Das Gefäßimplantat gemäß dieser Ausführungsform ist insbesondere dazu geeignet, bei einem Bauchaortenaneurysma 27 eingesetzt zu werden, wobei im implantierten Zustand der zylindrische Abschnitt 11-2 stromaufwärts des Aneurysmas 29 in der Bauchschlagader 27' fixiert sowie gegenüber der Gefäßwand zumindest im Übergangsbereich zu den Eintrittsöffnungen 13 weitgehend abgedichtet ist und die beiden Körper 11 mit ihren Austrittsöffnungen 15 jeweils in einer Beinschlagader 27" fixiert oder unfixiert enden.

### Bezugszeichenliste

- 11: Körper
- 13: Eintrittsöffnung
- 15: Austrittsöffnung
- 17: Wand
- 19: Durchgangskanal
- 21: Fixiermittel
- 23: Endpunkt
- 25: Kreislinie
- 27: Blutgefäß
- 29: Aneurysma
- 31: Flussrichtung
- 33: Pfeil
- 35: Pfeil
- 37: Abzweigung

## Patentansprüche

1. Gefäßimplantat zur Behandlung eines Aneurysmas, d.h. einer Aufweitung (29) der Querschnittsfläche eines Blutgefäßes (27), mit einem länglichen Körper (11), der eine Eintrittsöffnung (13), eine Austrittsöffnung (15) sowie einen die Eintrittsöffnung (13) mit der Austrittsöffnung (15) verbindenden Durchgangskanal (19) für durch das Blutgefäß (27) strömendes Blut aufweist, wobei der Durchgangskanal (19) in Umfangsrichtung durch eine blutundurchlässige Wand (17) begrenzt ist, wobei die Eintrittsöffnung (13) eine größere Querschnittsfläche besitzt als die Austrittsöffnung (15),
**dadurch gekennzeichnet,**
**dass** der Körper (11) mit blutdurchlässigen Fixiermitteln (21) für das austrittsöffnungsseitige Ende des Körpers (11) versehen ist, welche zur Anlage an der Gefäßwand des Blutgefäßes (27) ausgebildet sind, wobei die Fixiermittel (21) dazu ausgebildet sind, den Körper (11) im Bereich der Austrittsöffnung (15) von der Gefäßwand des Blutgefäßes (27) beabstandet zu halten.

2. Gefäßimplantat nach Anspruch 1,
**dadurch gekennzeichnet,**
**dass** sich der Körper (11), ein zwischen der Eintrittsöffnung (13) und der Austrittsöffnung (15) befindlicher Abschnitt (11-1) des Körpers (11) und/oder der Durchgangskanal (19) von der Eintrittsöffnung (13) zur Austrittsöffnung (15) hin verjüngt.

3. Gefäßimplantat nach Anspruch 2,
**dadurch gekennzeichnet,**
**dass** die Verjüngung gleichmäßig, kontinuierlich und/oder konisch ausgebildet ist.

4. Gefäßimplantat nach einem der vorstehenden Ansprüche,
**dadurch gekennzeichnet,**
**dass** der Körper (11) im Bereich der Eintrittsöffnung (13) und/oder an einem an die Eintrittsöffnung (13) anschließenden Abschnitt (11-2) des Körpers (11) zur Anlage an der Gefäßwand des Blutgefäßes (27) ausgebildet ist.

5. Gefäßimplantat nach nach Anspruch 4,
**dadurch gekennzeichnet,**
**dass** der an die Eintrittsöffnung (13) anschließende Abschnitt (11-2) des Körpers (11) zylinderförmig ausgebildet ist.

6. Gefäßimplantat nach Anspruch 4 oder 5,
**dadurch gekennzeichnet,**
**dass** der an die Eintrittsöffnung (13) anschließende Abschnitt (11-2) des Körpers (11) eine blutdurchlässig ausgebildete Wand besitzt.

7. Gefäßimplantat nach einem der vorstehenden Ansprüche,
**dadurch gekennzeichnet,**
**dass** die äußere Abmessung des Körpers (11) im Bereich der Austrittsöffnung (15) derart dimensioniert ist, dass der Körper (11) im Bereich der Austrittsöffnung (15) von der Gefäßwand des Blutgefäßes (27) beabstandet ist.

8. Gefäßimplantat nach einem der vorstehenden Ansprüche,
**dadurch gekennzeichnet,**
**dass** die Fixiermittel (21) im Bereich der Austrittsöffnung (15) an dem Körper (11) vorgesehen sind.

9. Gefäßimplantat nach einem der vorstehenden Ansprüche,
**dadurch gekennzeichnet,**
**dass** die Fixiermittel (21) als ein Gittergeflecht, mehrere jeweils mit einer radialen Komponente von dem Körper (11) abstehende Fixierelemente und/oder ein konischer Stent ausgebildet sind.

10. Gefäßimplantat nach einem der vorstehenden Ansprüche,
**dadurch gekennzeichnet,**
**dass** der Körper (11) oder ein Teil hiervon mit den Fixiermitteln (21) einteilig ausgebildet ist.

11. Gefäßimplantat nach einem der vorstehenden Ansprüche,
**dadurch gekennzeichnet,**
**dass** die Wand (17) des Körpers (11) starr, flexibel oder teils starr und teils flexibel ist und/ oder dass das Gefäßimplantat, insbesondere der Körper (11) oder die Wand (17) des Körpers (11), mehrteilig aufgebaut ist.

12. Gefäßimplantat, mit zwei Körpern (11) jeweils gemäß einem der vorstehenden Ansprüche, wobei die Körper (11) an einer Abzweigung (37) des Gefäßimplantats im Bereich ihrer Eingangsöffnungen (13) miteinander verbunden sind.

13. Gefäßimplantat nach Anspruch 12,
**dadurch gekennzeichnet,**
**dass** die beiden Körper (11) einen an die Abzweigung (37) anschließenden gemeinsamen Abschnitt (11-2) zur Anlage an der Gefäßwand des Blutgefäßes (27) aufweisen.

## Claims

1. A vessel implant for the treatment of an aneurysm, i.e. a dilatation (29) of the cross-sectional area of a blood vessel (27), comprising an elongate body (11) which has an inlet opening (13), an outlet opening (15) and a passage (19) connecting the inlet opening (13) to the outlet opening (15) for blood flowing through the blood vessel (27), wherein the passage (19) is bounded in the peripheral direction by a blood-impermeable wall (17), and wherein the inlet opening (13) has a larger cross-sectional area than the outlet opening (15),
**characterized in that**
the body (11) is provided with blood-permeable fixing means (21) for the end of the body (11) at the outlet opening side, said fixing means being designed for contact with the vessel wall of the blood vessel (27), with the fixing means (21) being designed to hold the body (11) spaced apart from the vessel wall of the blood vessel (27) in the region of the outlet opening (15).

2. A vessel implant in accordance with claim 1,
**characterized in that**
the body (11), a section (11-1) of the body (11) located between the inlet opening (13) and the outlet opening (15) and/or the passage (19) converges/converge from the inlet opening (13) toward the outlet opening (15).

3. A vessel implant in accordance with claim 2,
**characterized in that**
the convergence is uniform, continuous and/or conical.

4. A vessel implant in accordance with any one of the preceding claims,
**characterized in that**
the body (11) is designed for contact with the vessel wall of the blood vessel (27) in the region of the inlet opening (13) and/or at a section (11-2) of the body (11) adjoining the inlet opening (13).

5. A vessel implant in accordance with claim 4,
**characterized in that**
the section (11-2) of the body (11) adjoining the inlet opening (13) is cylindrical.

6. A vessel implant in accordance with claim 4 or claim 5,
**characterized in that**
the section (11-2) of the body (11) adjoining the inlet opening (13) has a blood permeable wall.

7. A vessel implant in accordance with any one of the preceding claims,
**characterized in that**
the outer dimension of the body (11) is dimensioned in the region of the outlet opening (15) such that the body (11) is spaced apart from the vessel wall of the blood vessel (27) in the region of the outlet opening (15).

8. A vessel implant in accordance with any one of the preceding claims,
**characterized in that**
the fixing means (21) are provided at the body (11) in the region of the outlet opening (15).

9. A vessel implant in accordance with any one of the preceding claims,
**characterized in that**
the fixing means (21) are made as a mesh, as a plurality of fixing elements respectively projecting from the body (11) with a radial component and/or as a conical stent.

10. A vessel implant in accordance with any one of the preceding claims,
**characterized in that**
the body (11) or a part thereof is made in one piece with the fixing means (21).

11. A vessel implant in accordance with any one of the preceding claims,
**characterized in that**
the wall (17) of the body (11) is rigid, flexible or partly rigid and partly flexible; and/or **in that** the vessel implant, in particular the body (11) or the wall (17) of the body (11), is made in multiple parts.

12. A vessel implant, comprising two bodies (11), each in accordance with any one of the above claims, wherein the bodies (11) are connected to one another at a branch (37) of the vessel implant in the region of their respective inlet openings (13).

13. A vessel implant in accordance with claim 12,
**characterized in that**
the two bodies (11) have a common section (11-2) adjoining the branch (37) for contact with the vessel wall of the blood vessel (27).

## Revendications

1. Implant vasculaire pour le traitement d'un anévrisme, c'est-à-dire d'un élargissement (29) de l'aire de section transversale d'un vaisseau sanguin (27), comprenant un corps allongé (11) qui comporte une ouverture d'entrée (13), une ouverture de sortie (15) ainsi qu'un canal traversant (19) reliant l'ouverture d'entrée (13) à l'ouverture de sortie (15) pour le sang qui s'écoule à travers le vaisseau sanguin (27), dans lequel le canal traversant (19) est limité en direction périphérique par une paroi imperméable au sang (17), et l'ouverture d'entrée (13) possède une aire de section transversale plus grande que l'ouverture de sortie (15),
**caractérisé en ce que**
le corps (11) est doté de moyens de fixation (21) perméables au sang pour l'extrémité du corps (11) du côté de l'ouverture de sortie, ces moyens étant réalisés pour venir s'appliquer contre la paroi du vaisseau sanguin (27), et les moyens de fixation (21) sont réalisés pour maintenir le corps (11) à distance de la paroi du vaisseau sanguin (27) dans la région de l'ouverture de sortie (15).

2. Implant vasculaire selon la revendication 1,
**caractérisé en ce que** le corps (1), un tronçon (11-1) du corps (11) qui se trouve entre l'ouverture d'entrée (13) et l'ouverture de sortie (15) et/ou le canal traversant (19) va en se rétrécissant de l'ouverture d'entrée (13) vers l'ouverture de sortie (15).

3. Implant vasculaire selon la revendication 2,
**caractérisé en ce que** le rétrécissement est réalisé de manière régulière, en continu et/ou conique.

4. Implant vasculaire selon l'une des revendications précédentes,
**caractérisé en ce que** le corps est réalisé, dans la région de l'ouverture d'entrée (13) et/ou au niveau d'un tronçon (11-2) du corps (11) qui fait suite à l'ouverture d'entrée (13), pour venir s'appliquer contre la paroi du vaisseau sanguin (27).

5. Implant vasculaire selon la revendication 4,
**caractérisé en ce que** le tronçon (11-2) du corps (11) qui fait suite à l'ouverture d'entrée (13) est réalisé sous forme cylindrique.

6. Implant vasculaire selon la revendication 4 ou 5,
**caractérisé en ce que** le tronçon (11-2) du corps (11) qui fait suite à l'ouverture d'entrée (13) possède une paroi réalisée perméable au sang.

7. Implant vasculaire selon l'une des revendications précédentes,
**caractérisé en ce que** la dimension extérieure du corps (11) est choisie, dans la région de l'ouverture de sortie (15), de telle façon que le corps (11) est écarté de la paroi du vaisseau sanguin (27) dans la région de l'ouverture de sortie (15).

8. Implant vasculaire selon l'une des revendications précédentes,
**caractérisé en ce que** les moyens de fixation (21) sont prévus sur le corps (11) dans la région de l'ouverture de sortie (15).

9. Implant vasculaire selon l'une des revendications précédentes,
**caractérisé en ce que** les moyens de fixation (21) sont réalisés sous la forme d'un treillis grillagé, de plusieurs éléments de fixation dépassant du corps (11) chacun avec une composante radiale et/ou d'un stent conique.

10. Implant vasculaire selon l'une des revendications précédentes,
**caractérisé en ce que** le corps (11) ou une partie de celui-ci est réalisé(e) d'un seul tenant avec les moyens de fixation (21).

11. Implant vasculaire selon l'une des revendications précédentes,
**caractérisé en ce que** la paroi (17) du corps (11) est rigide, flexible ou partiellement rigide et partiellement flexible, et/ou **en ce que** l'implant vasculaire, en particulier le corps (11) ou la paroi (17) du corps (11), est constitué en plusieurs parties.

12. Implant vasculaire comprenant deux corps (11) respectivement selon l'une des revendications précédentes, dans lequel les corps (11) sont reliés l'un à l'autre au niveau d'une ramification (17) de l'implant vasculaire dans la région de leurs ouvertures d'entrée (13).

13. Implant vasculaire selon la revendication 12,
**caractérisé en ce que** les deux corps (11) comprennent un tronçon commun (11-2) qui fait suite à la ramification (37), pour venir s'appliquer contre la paroi du vaisseau sanguin (27).
